# EUROPEAN PATENT SPECIFICATION

(11) **EP 1 120 094 B1**
(45) Date of publication and mention of the grant of the patent: **21.06.2006**
(21) Application number: 00610013.5
(22) Date of filing: 28.01.2000
(51) Int. Cl.: A61F 2/06, A61M 25/00

(54) **A delivery catheter for a self-expandable prosthesis**
Einführkatheter für eine selbstausdehnbare Prothese
Cathéter pour une prothèse auto-expansible

(43) Date of publication of application: 01.08.2001
(73) Proprietor: WILLIAM COOK EUROPE ApS, 4632 Bjaeverskov (DK); Cook Incorporated, Bloomington, IN 47402-0489 (US)
(72) Inventor: Klint, Henrik Sonderskov, 2800 Lyngby (DK)
(74) Representative: Indahl, Peter Jensen

(56) References cited:
- WO-A-93/04722
- DE-A- 3 641 935
- US-A- 5 178 158
- US-A- 5 643 278
- US-A- 5 957 940

## Description

The present invention relates to a delivery system comprising a delivery device with a distal end and a shaft portion with a lumen extending in the longitudinal direction from a proximal end towards the distal end, a self-expandable prosthesis arranged in a prosthesis receptacle at the distal end of the delivery device, and a pusher member arranged in the lumen of said delivery device.

A radially compressible and self-expandable prosthesis, such as a stent, a stent-graft, a valve member, or a filter, is biased to expand and anchor within a body lumen to support the wall of the lumen and/or to dilate the wall outwardly. Stents are applied in lumens in a body, including blood vessels, urinary tracts, bile duct ect. Self-expanding stents are commonly delivered to a desired location in a lumen using a catheter, in which the stent is constrained in a radially compressed configuration. In the loaded state with a self-expanding stent tightly compressed within the catheter, the stent imposes significant forces against the surrounding catheter body.

A delivery system of this type is known from EP-A 812 579 to Nitinol Devices and Components Inc. whose delivery device is a traditional catheter provided with a tubular member at its distal end to accomodate a radially compressed stent. The tubular member is formed from a shape memory alloy, the wall of tubular member being partially slotted to facilitate flexing of the member in bending deformation. When the delivery system has been manoeuvred to the desired location, the stent is discharged by application of a pushing force against the proximal end of the stent relative to the catheter and the tubular member, e.g. by means of a rod with a plunger on its distal end. The catheter flexes when submitted to torque, and the slots in the tubular member can clutch the compressed stent when it is pushed forward for release.

Another delivery system is known from EP-A 696 447 where a prosthesis is carried over the distal end of a shaft where it is contained in a radially compressed configuration by a sheath. After introducing the catheter with the stent to a desired target location within a body lumen, the prosthesis may be released by proximally retracting the sheath. This catheter is also flexible to torque, and the sheath has only limited flexibility due to its large diameter.

It is an object of the present invention to provide a delivery system with a delivery device which in its distal area is very flexible and yet capable of transferring torque.

In view of this, the delivery system according to the invention is characterized in that the shaft portion of said delivery device is made of a first multifilar row of at least two helically wound wires placed next to each other capable of transferring torque to the distal end of the delivery device .

By making the shaft of a row of two or more wires which row is helically wound with a pitch roughly corresponding to the width of the adjacent wires in the row, the wound wires transfer torque and also force components directed in the axial direction of the delivery device to the distal end thereof, and this construction is found to give a very high resistance to kinking of the delivery device. When the shaft is heavily bent its cross-section maintains a circular shape, and the forces transmitted through the helically wound wires have less tendency to be concentrated in the area of the bend. This is a distinct advantage over prior art delivery systems in which the shafts are deformed into oval shape when bent, and thus they are much more prone to kinking. The delivery device surprisingly maintains its capabilities for transferring torque and push when it follows a tortuous path involving two or more loops, probably because of the good kinking resistance.

Due to the very high flexibility, pushability and torqueability and the ability of the construction to maintain each of these three characteristics even when set in a very tortuous pattern involving two or more tight loops, the delivery system can be of use in very small and distant vessels. The delivery system can even be used without the need of a separate guide wire because the shaft of the delivery device can be manoeuvred to the desired prosthesis deployment site like a guidewire, and by dispensing with the guide wire the flexibility of the delivery system is further increased.

In one embodiment the said prosthesis receptacle is fixed to said first helically wound multifilar row of wires in axial extension thereof. This allows the prosthesis receptacle to be designed and manufactured independently of the shaft portion. The mounting in direct extension of the row of wires makes the prosthesis receptacle follow torsional actions on the shaft portion.

Although the prosthesis receptacle can be designed in any manner capable of resisting the outward pressure applied to the inside of the prosthesis receptacle by the compressed prosthesis, it is preferred that the prosthesis receptacle is a tubular segment of multifilar wound construction, such as a braided wire construction providing the prosthesis receptacle with a high flexibility. More preferably the prosthesis receptacle is a construction of a second helically wound multifilar row of wires. The latter design makes it possible to obtain a very diminutive outer diameter as only a single layer of wires is required.

In another embodiment the prosthesis receptacle is a distal segment of said first helically wound multifilar row of wires. This integrated design is very simple to manufacture because the self-expanding prosthesis is simply loaded into the distal end portion of the shaft portion, and very safe to use because a minimum number of parts are used.

In applications where it is desirable to have a delivery system of relatively small outer dimensions it is preferred that the prosthesis receptacle has a larger lumen than the lumen of said shaft portion. The wire or wall thickness required for resisting the outward pressure from the radially compacted prosthesis is smaller than the wire thickness required to transmit axial thrust (pushability) over a long shaft distance, such as 80 cm or more, and by providing the shaft portion with the smallest lumen it can have the largest wall thickness within a given outer diameter. The receptacle thus has a lesser wall thickness than the shaft portion, but the same outer diameter.

In an further embodiment, which is an alternative to using a traditional rod-like pusher with a solid shaft, the pusher member has a shaft made of a third helically wound multifilar row of wires which makes the pusher member a hollow construction with torqueability and pushability similar to the shaft portion of the delivery device and with slightly larger flexibility due to the smaller outer diameter of said third row of wires.

In yet another embodiment the wires in said first row of wires and possibly also in said second or third row of wires have a pitch angle in the range of 35°-76°, preferably a pitch angle in the range of 40°-70°. Although it is possible to use other pitch angles, angles chosen in these ranges provide a balanced solution to the requirements for the desired high flexibility, good pushability and fine torqueability. The inner range of 40°-70° is in particular useful for placement of a prosthesis in very distant, small sized vessels, such as in blood vessels in the brain, whereas the subrange of 35°-40° is applicable when very high flexibility is a requirement, and the subrange of 70°-76° is applicable when very high pushability is a requirement. It is of course possible to choose different pitch angles in different segments of the individual row of wires and/or to choose different pitch angles for the different rows of wires.

In a further embodiment the row or rows of wires is/are made up of one or more groups of from 2 to 12 helically wound wires, preferably of from 4 to 8 helically wound wires. By using several wires their aggregate width can be adapted to correspond to the desired pitch for the given diameter of introducer device. Groups of more than 12 wires have a tendency to buckle when they are helically wound in a common winding operation. For wires of round cross-sectional shape a number of from 4 to 8 wires per group is preferred, but for flat wires or wires of oval shape two or three wires per group can be more suitable.

In order to promote uniform and well-defined characteristics of the introducer system along its length, the wires in a group can be located closely next to each other so that they mutually support each other. In this manner a possible deflection of a single wire strand is reduced to a minimum by the other wires in the group. As the wires in the group are wound into a helical course in a common movement there can be a interstice between the turns of the group of wires.

It is possible to promote the flexibility of the introducer device by machining the wires in said first row to a lesser outer diameter, e.g. by grinding, at a region of the delivery device. The region can extend along the whole length of the shaft portion of the introducer device, so that it is given a very precise outer dimension by the machining. In another embodiment the region is a distal region machined to a tapering shape with decreasing outer diameter in the distal direction causing the device to have an increasing flexibility towards the distal end which promotes introduction into very diminutive vessels. The reduced cross-sectional area of the wires produced by the machining greatly increases the bending flexibility of the delivery device without sacrificing its ability to transfer torque.

In a preferred embodiment, the distal tip of the delivery system is provided with marker means for making it radiopaque, e.g. by a gold or platinum plating, soldering, brazing or by laser welding a radiopaque member onto the distal tip. The marker promotes precise positioning of the prosthesis.

In order to facilitate mutual movement of the parts in the introducer system during release of the prosthesis the pusher member can be provided with a coating to decrease friction between the pusher member and the delivery device, and/or the inner surface of the delivery device shaft portion can be provided with a coating to decrease friction and/or the inner surface of the prosthesis receptacle can be provided with such a coating.

It is preferred that the delivery system at least in a 30 cm long distal area has a maximum outer diameter of less than 3.0 mm, and suitably less than 2.0 mm. An maximum diameter of 3 mm in the part of the delivery system advanced through the vascular system allows for a straightforward percutaneous introduction by the Seldinger technique and easy navigation through the curves in the larger vessels. As use of a traditional, separate sheet for keeping the prosthesis compressed can be wholly dispensed with because the prosthesis receptacle is in itself capable of keeping the prosthesis in the fully compressed state, the outer diameter of the receptacle and the shaft portion is identical to the maximum outer diameter of the delivery system portion introduced into the vascular system. Consequently, the maximum outer diameter can be kept less than 2.0 mm and even less than 1.00 mm, which allows introduction into quite fine and diminutive vessels such as into the external and internal carotid arteries. It is further possible to restrict the maximum outer diameter to at the most 0.75 mm which makes it possible to advance the prosthesis into e.g. the liver or other soft tissue areas, and by keeping the maximum outer diameter below 0.30 mm in a distal end area having a length of at least 10 cm even the most distant vascular regions become accessible.

In a further embodiment the number of wires in said first helically wound row of wires varies along the length of the delivery device. This can be attained by reducing, during the winding operation, the number of wires in the row. The lower number of wires in the row make it possible to wind the remaining wires with a larger pitch angle which increases the flexibility of the device. It is preferred that the number of wires diminishes in the distal direction so that the softness of the delivery device increases without any change of material and without bonding together several separate device segments.

When the delivery device has to traverse large lumen vascular paths in order to reach the more difficult small size vascular vessels, the first helically wound row of wires can be stiffened in a proximal segment of said shaft by a supplementary tubular member, such as a cannula.

In the following, examples of the invention are descriped in more detail with reference to the very schematic drawings, on which
Fig. 1 is a side view of the an embodiment of the delivery system according to the present invention,
Figs. 2-6 are enlarged partial views in longitudinal section of various embodiments of the delivery system in Fig. 1,
Figs. 7 and 8 are partial views in longitudinal section of two further embodiments,
Fig. 9 is an illustration of the delivery system in position in the vascular system during release of the prosthesis, and
Fig. 10 depicts a winding operation on a multifilar row of wires.

In the following description of the depicted embodiments the same reference numerals are used for features of the same type.

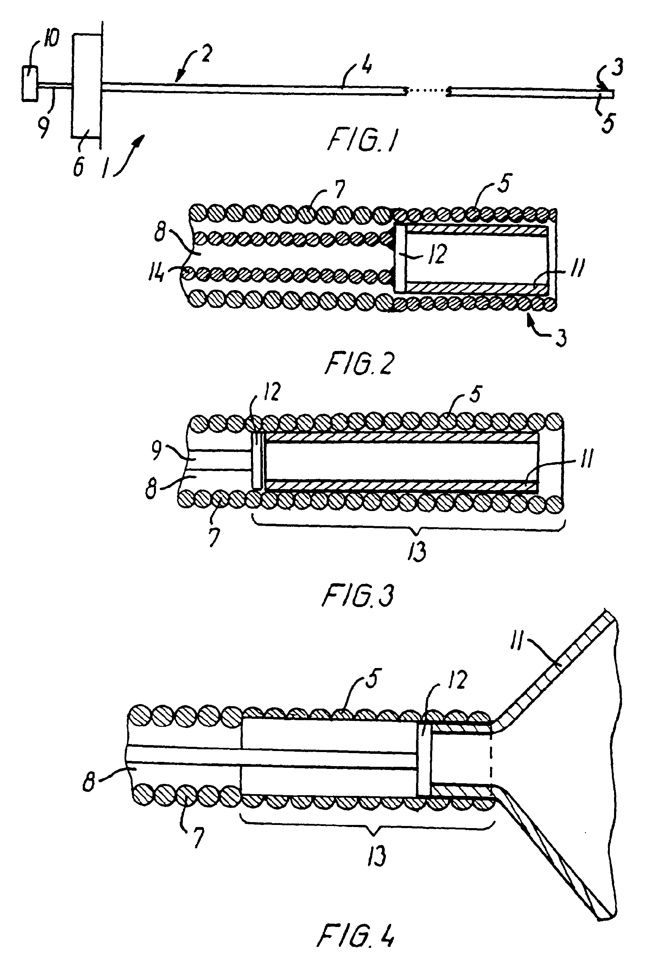

A delivery system depicted in Fig. 1 is generally denoted 1 and includes a delivery device 2 having a distal end 3 and a shaft portion 4 extending between a prosthesis receptacle 5 at the distal end and a proximal mounting member 6 fixedly mounted to the shaft portion. The shaft portion is made of a first helically wound multifilar row of wires 7 and it has a central longitudinally extending lumen 8.

The delivery system further comprises a pusher member 9 which can be inserted through the lumen 8. A handle or pin vice 10 is mounted on the pusher member for pushing it forward in the distal direction when a prosthesis 11 located in receptacle 5 is to be released from the introducer device by being pushed out of receptacle 5. Pin vice 10 and mounting member 6 can be parts of a unitary control device to be manually actuated when the prosthesis has been introduced and positioned at the desired vascular site.

At the distal end of the pusher member 9 an engagement means 12 can act on the prosthesis 11. The engagement means can e.g. be a plate of a dimension fitting into receptacle 5 and abutting the proximal end of the prosthesis so that the plate pushes the prosthesis out of the receptacle when the pusher member is pushed forwards. The engagement means can also be designed as an elongate member that extends coaxially inside the radially compressed prosthesis and engages the prosthesis at several locations along the length thereof so that the prosthesis is partly pulled, partly pushed out of the receptacle. These engagement points or areas can be effected by radial projections, hooks, ridges, or another kind of engagement means such as a high friction material. This can be an advantage if the prosthesis has an extensive length, and in particular if it has a construction having a tendency to buckle when pushed upon.

By the term "prosthesis receptacle" is meant any structure or region near or at the distal end of a delivery device where a radially compressible tubular prosthesis is carried during manoeuvring of the delivery device and prosthesis within a body lumen. The prosthesis receptacle 5 can be made of a length of tubular material that is flexible in itself or is made flexible by incisions or due to its construction, such as a construction of wound or braided wires. If the prosthesis is rather short in length or is for deployment in a large sized vessel of a rather straight shape, such as in the aorta, the receptacle need not be flexible and can be made out of a stiff tubular member.

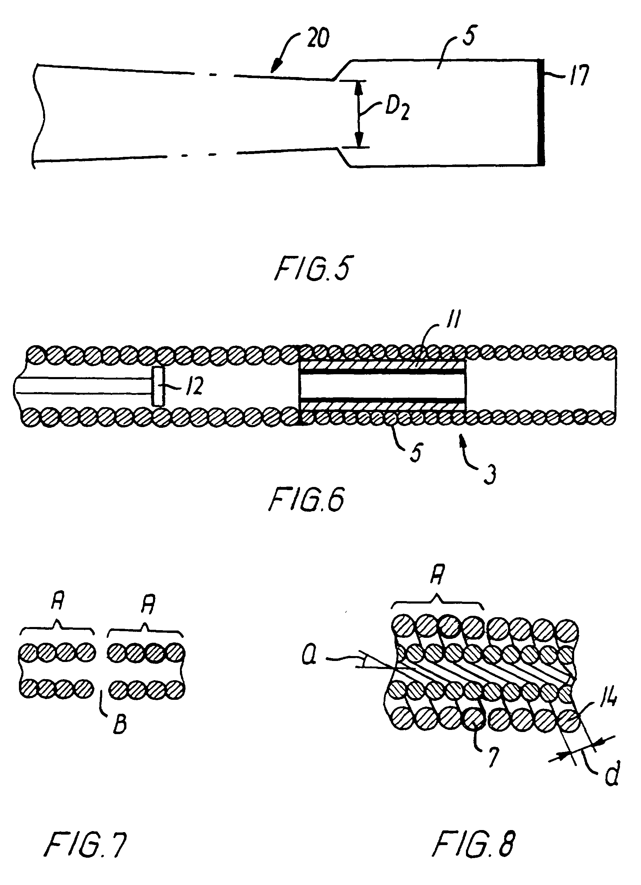

The length of the prosthesis receptacle is at least of the same size as the length of the loaded prosthesis. However, other lengths are also possible. As depicted in Fig. 6 the receptacle can have a length that is considerably longer than the loaded prosthesis, so that the prosthesis can be loaded into a position at the proximal end of the receptacle leaving empty a distal length of the receptacle. This free distal length will not be stiffened by the presence of the loaded prosthesis and will consequently be very soft and flexible. The length can e. g. be chosen so that the free distal length is in the range of 5-150 mm, preferably in the range of 10-50 mm.

In a preferred embodiment the prosthesis receptacle 5 is made of a second helically wound multifilar row of wires. As depicted in Fig. 2 the second row of wires can be made independently of the first row of wires and in different dimensions or different materials than the first row of wires, and the receptacle 5 is then fixed in axial extension of the first row of wires, e.g. by laser welding, soldering, bracing, or mechanical locking, such as press-fitting into the lumen of the shaft portion, or binding with threads or suture. An alternative embodiment is depicted in Figs. 3 and 4 where prosthesis receptacle 5 is made integral with shaft portion 4 by using a distal segment 13 of said first row of wires 7 as the receptacle.

In the embodiment of Fig. 3 the inner lumens in the shaft portion and in receptacle 5 are of even size which brings the advantages of being able to load prostheses of various lengths in one and the same delivery system and of being able to load from the proximal end of the delivery device a pusher member having a solid engagement means 12 of a diameter that is only slightly less than the diameter of the inner lumen 8.

In the embodiment of Fig. 2 the pusher member is inserted from the distal end of the shaft portion prior to loading the prosthesis into receptacle 5. This allows the engagement means 12 to be of a larger diameter than the lumen 8 of shaft portion 4.

In the embodiment of Fig. 6 the radially compacted prosthesis projects radially inwards beyond the step in inner lumen diamter at the transition between receptacle 5 and shaft portion 4. Consequently it is possible to use a pusher member 9 having an engagement member 12 of less diameter than lumen 8 and yet push the prosthesis out of receptacle 5 by pressing against the proximal end of the prosthesis.

The shaft of the pusher member 9 can be of a small diameter solid wire or rod as depicted in Fig. 3 or it can be made of a third helically wound multifilar row of wires 14 as depicted in Fig. 2.

The receptacle in the embodiment of Fig. 4 is made by machining the inside of the wound wires to a larger lumen. This can e. g. be done by spark erosion or grinding. In the latter case the distal end portion of the wound wires are placed in a retaining ring that is longitudinally displaceable with respect to a coaxilly mounted grinding wheel.

A grinding procedure can also be used to produce a tapered section 20 in shaft portion 4 (sketched in Fig. 5). The taper can extend along a substantial length of the shaft portion. In the tapered section the outer diameter of the delivery device diminishes to diameter D2. Due to the taper the delivery device obtains a gradually increasing transverse flexibility and a higher softness, but torque is nevertheless surprisingly transferred fully to the receptacle. As an alternative or supplement to grinding the shaft portion can be composed of several wire portions in which the wires have mutually different diameters and cross-sectional areas.

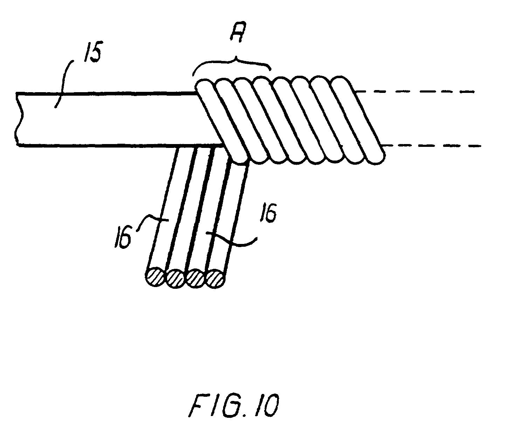

The wires used in the first, second and/or third helically wound multifilar row of wires are of a linear elastic material, such as stainless steel, titanium or tantalum, or it is made of a superelastic alloy, such as Nitinol. Preferably, the wire has an ultimate tensile strength in the range of 1800-2700 N/mm² but lower or higher values are also possible. The construction (shaft portion 4 of the delivery device, the shaft of pusher member 9 and/or receptacle 5) is made by placing a group of from two to twelve wires in a row next to each other, e.g. according to the desired pitch angle, whereafter the group of wires is wound about a mandrel 15. Because a row of wires is wound, the individual wire is restricted in movement by the other wires and is plastically deformed into a permanent helical shape which is kept without any further restraints than the remaining wires in the row. The winding can be done on the inside end of a tubular support member where the row of wires are inserted at said end by rotating and simultaneously pushing the wires against the inside of the support. The wound wire then exits at the other end of the support. This produces a shaft with a very precise outer diameter. Alternatively, the winding operation can take place about a mandrel 7 as in Fig. 10 which depicts winding of a row A of four identical wires 16. After the winding the mandrel with the coiled wires can be subjected to heat treatment in order to remove residual stresses from the wires. As an example the heat treatment can last for about two hours in an oven at a temperature of about 500°C. Generally, the temperature can be in the range of 400-600°C and the holding time at the temperature can last for many hours, such as up till 20 hours or more. After the heat treatment the mandrel is removed from the wires. The resulting helically wound multifilar row of wires maintain their mutual position even when heavily torqued, bent or pushed, presumably because each single wire is supported by the contiguous wires in the row. The winding operation can be effected so that the windings are touching each other, but preferably it is performed so that an interstice B is present between the turns (Fig. 7). The interstice facilitates bending of the shaft portion in thight turns.

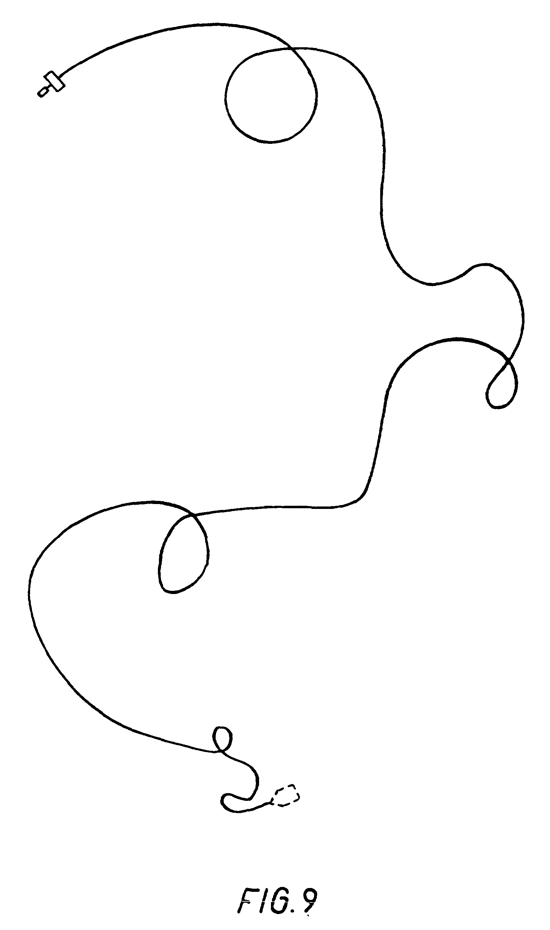

The size of the pitch angle a (Fig. 8) depends on the diameter of the wires, the diameter of the construction and the number of wires in the row. The most preferred pitch angle a for the delivery device and push member is in the range of 40°-65° or 50° to 70°. However, the combination of torque transferral, pushability and transverse flexibility is normally well-balanced for pitch angles in the range of 50-68°. The diameter d of the wire is typically in the range of 0.06-0.75 mm, and preferably in the range of 0.15-0.45 mm.

In order to make the tip portion of the delivery system more visible on a screen, it is desirable to use some kind of radiopaque material, such as platinum or gold. In the embodiment illustrated in Fig. 5, the terminal end of the distal tip 3 of the delivery system is provided with a marker means 17 for making it radiopaque, e.g. a gold layer or a gold thread.

The delivery device can be made with uniform diameter throughout its length. In case the delivery device has diminishing diameter towards the distal end a prefabricated delivery device of uniform diameter can be grinded to the desired dimensions.

As an alternative or supplement to grinding the delivery device can be composed of several wire portions in which the wires have mutually different diameters and cross-sectional areas. In a proximal portion the wires can have a larger diameter than the wires in a distal portion.

In the follwing some examples of delivery systems made according to the invention are described.

### EXAMPLE 1:

A delivery device was made of a first helically wound row of 4 wires of 0.35 mm wire diameter d. The shaft of wound wires had initially an outside diameter of 1.67 mm and an inner lumen of 0.97 mm. The receptacle was made up of a second helically wound row of four wires of 0.20 mm wire diameter. The receptacle had a length of 37 mm and initially an outside diameter of 1.70 mm and an inside diameter of 1.3 mm. A radially compressed stent was arranged inside the receptacle. The loaded stent had a length of 35 mm and was recessed a little in relation to the distal end of the receptacle. The pusher member was made of a third helically wound row of four wires of 0.28 mm wire diameter and a shaft outer diameter of 0.91 mm. A plunger element or an engagement member was located on the distal end of the shaft. The shaft and the receptacle of the delivery device was ground to an common outer diameter of 1.5 mm (4.5 French). In its fully self-expanded state the stent had an outer diameter of 8 mm. The delivery device was set in a complex curved shape involving three consecutive loops of a loop diameter of 20 mm axially separated by two loops of a loop diameter of 15 mm and a number of further turns representative of a complex vascular structure. Then the shaft of the delivery device was manipulated and it proved to be easily pushed forward and retracted as well as easily torqued. Then the pusher member was pushed forward in relation to the shaft portion, and the stent was easily pushed out of the receptacle without causing noticeable flexion or movement of the delivery device.

### EXAMPLE 2:

A delivery device was made of a first helically wound row of 5 wires of 0.30 mm wire diameter d. The winding of the shaft was made with an outside diameter of 1.20 mm and an inner lumen of 0.6 mm. The receptacle was made up of a second helically wound row of four wires of 0.15 mm wire diameter. The receptacle had a length of 60 mm and an outside diameter of 1.20 mm and an inside diameter of 0.9 mm. A radially compressed prosthesis was arranged inside the receptacle. The loaded prosthesis had a length of 20 mm and was positioned at the proximal end of the receptacle with a 40 mm very soft free distal receptacle end. The pusher member was made of a single 0.35 mm diameter wire rod that carried an engagement member at its distal end. In its fully self-expanded state the prosthesis had an outer diameter of 3 mm. The delivery device was advanced through a complex curved vascular system involving several consecutive, retrograde turns in vessels having a lumen of only 2 mm or less. Then the pusher member was pushed forward in relation to the shaft portion, and the stent was easily and well-controlled pushed out of the receptacle.

### EXAMPLE 3:

A combined receptacle and distal shaft segment of a delivery device was made of a first helically wound row of 8 wires of 0.075 mm wire diameter d. The winding was 5 made with an outside diameter of 0.25 mm and an inner lumen of 0.1 mm. The combined receptacle and distal shaft segment had a length of 12 cm. A prosthesis was compressed radially to an outer diameter of 0.07 mm and was pushed into the receptacle. The loaded prosthesis had a length of 10 mm and was positioned in the receptacle with its proximal end 25 mm from the distal receptacle end. The pusher member was made of a single 0.08 mm diameter solid wire rod. The pusher member was used to push the stent out of the receptacle.

When the delivery system is to be introduced into the vascular system there is firstly established a percutaneous puncture site, e.g. by the Seldinger technique or an existing puncture site is used. Then the shaft portion of the delivery device is inserted through the cannula, sheat or hemostatic valve at the puncture site and the delivery system is advanced and navigated through the vascular system to the prosthesis deployment site. Due to the very high flexibility, pushability and torqueability of the delivery system it can be advanced to the prosthesis deployment site without use of a guidewire, a catheter or a sheat to negotiate the sharp curves in the path. When the prosthesis receptacle is at the desired position the pusher member is activated and the prosthesis is pushed out to expand as indicated in Figs. 4 or 9.

For some applications it is desirable to deploy a prosthesis that has been provided with an active substance, such as a cell growth inhibitor. The active substance can have such a short shelf life that it needs to be applied to the prosthesis immediately prior to deploying the prosthesis. This can be done by dipping the distal end of the delivery device, viz. the prosthesis in the receptacle, into a fluid of active substance.

Individual features of the various embodiments can be combined into further embodiments according to the present invention.

## Claims

1. A delivery system (1) comprising a delivery device (2) with a distal end (3) and a shaft portion (4) with a lumen (8) extending in the longitudinal direction from a proximal end towards the distal end (3), a self-expandable prosthesis (11) arranged in a prosthesis receptacle (5) at the distal end of the delivery device, and a pusher member (9) arranged in the lumen (8) of said delivery device, **characterized in that** the shaft portion (4) of said delivery device is made of a first multifilar row of at least two helically wound wires (7) placed next to each other capable of transferring torque to the distal end (3) of the delivery device.

2. A delivery system according to claim 1, **characterized in that** said prosthesis receptacle (5) is fixed to said first helically wound multifilar row of wires (7) in axial extension thereof.

3. A delivery system according to claim 2, c h a r a c t e r i z e d in that the prosthesis receptacle (5) is a tubular segment of multifilar wound construction.

4. A delivery system according to claim 3, **characterized in that** the prosthesis receptacle (5) is a second helically wound multifilar row of wires.

5. A delivery system according to claim 1, **characterized in that** the prosthesis receptacle (5) is a distal segment (13) of said first helically wound multifilar row of wires. (7).

6. A delivery system according to any of claims 1 to 5, **characterized in that** the prosthesis receptacle (5) has a larger lumen than the lumen (8) of said shaft portion (4).

7. A delivery system according to any of claims 1 to 6, **characterized in that** the prosthesis receptacle (5) is longer than the prosthesis.

8. A delivery system according to any of claims 1 to 7, **characterized in that** the prosthesis receptacle (5) is 5-150 mm longer than the prosthesis (11).

9. A delivery system according to any of claims 1 to 8, **characterized in that** the pusher member (9) has a shaft made of a helically wound multifilar row of shaft wires (14).

10. A delivery system according to any of claims 1 to 9, **characterized in that** the wires in said first row of wires (7) and possibly also in said second row of wires or in said row of shaft wires (14) have a pitch angle in the range of 26°-76°.

11. A delivery system according to claim 10, **characterized in that** the wires in said first row of wires (7) and possibly also in said second row of wires or in said row of shaft wires (14) have a pitch angle in the range of 40°-65°.

12. A delivery system according to any of claims to 11, **charaeterized** in that the row or rows of wires (7, 14) is/are made up of one or more groups of from 4 to 8 helically wound wires.

13. A delivery system according to claim 12, **characterized in that** the wires in a group are located closely next to each other.

14. A delivery system according to any of claims 1 to 13, **characterized in that** the wires in said first row (7) have a lesser outer diameter at a region of the delivery device.

15. A delivery system according to claim 14, **characterized in that** said region is a distal region having a tapering shape with decreasing outer diameter in the distal direction.

16. A delivery system according to any one of the preceding claims, **characterized in that** the distal tip (3) of the delivery system is provided with a marker means (17) for making it radiopaque.

17. A delivery system according to any of claims 1 to 16, **characterized in that** the pusher member (9) is provided with a coating to decrease friction between the pusher member (1) and the delivery device, and/or the inner surface of the delivery device shaft portion (4) is provided with a coating to decrease friction and/or the inner surface of the prosthesis receptacle (5) is provided with such a coating.

18. A delivery system according to any of claims 1 to 17, **characterized in that** the delivery system at least in a 30 cm long distal area has a maximum outer diameter of less than 2.0 mm.

19. A delivery system according to any of claims 1 to 18, **characterized in that** the delivery system at least in a 30 cm long distal area has a maximum outer diameter of less than 1.00 mm.

20. A delivery system according to claim 18 or 19, **characterized in that** the delivery system in a distal end area having a length of at least 10 cm has a maximum outer diameter of less that 0.30 mm.

21. A delivery system according to any one of the preceeding claims, **characterized in that** the number of wires in said first helically wound row of wires (7) varies along the length of the delivery device.

22. A delivery system according to any of claims 4 to 21, **characterized in that** the number of wires in said second helically wound row of wires varies along the length of the delivery device.

23. A delivery system according to any one of the preceeding claims, **characterized in that** in a proximal segment of said shaft (4) the first helically wound row of wires (7) is stiffened by a supplementary tubular member.

## Patentansprüche

1. Einführsystem (1) bestehend aus einer Einführvorrichtung (2) mit einem Distalende (3) und einem Schaftteilstück (4) mit einem sich in Längsrichtung vom Proximalende zum Distalende (3) erstreckenden Lumen (8), einer in einem Prothesenbehälter (5) am Distalende des Einführsystems angeordneten selbstausdehnbaren Prothese (11) und einem im Lumen der Einführvorrichtung liegenden Druckglied (9), **dadurch gekennzeichnet, dass** das Schaftteilstück (4) der Einführvorrichtung aus einer ersten multifilen Reihe, bestehend aus zumindest zwei nebeneinander platzierten spiralförmig gewundenen Drähten, hergestellt ist, die geeignet sind, ein Drehmoment auf das Distalende (3) der Einführvorrichtung zu übertragen.

2. Einführsystem nach Anspruch 1, **dadurch gekennzeichnet, dass** der Prothesenbehälter (5) an der ersten spiralförmig gewunden multifilen Reihe an Drähten (7) in axialer Richtung befestigt ist.

3. Einführsystem nach Anspruch 2, **dadurch gekennzeichnet, dass** der Prothesenbehälter (5) ein rohrförmiges Segment des multifil gewunden Aufbaus ist.

4. Einführsystem nach Anspruch 3, **dadurch gekennzeichnet, dass** der Prothesenbehälter (5) eine zweite spiralförmig gewundene multifile Reihe an Drähten darstellt.

5. Einführsystem nach Anspruch 1, **dadurch gekennzeichnet, dass** der Prothesenbehälter (5) ein Distalsegment (13) der ersten spiralförmig gewundenen mutlifilen Reihe an Drähten (7) darstellt.

6. Einführsystem nach einem der Ansprüche 1 bis 5, **dadurch gekennzeichnet, dass** der Prothesenbehälter (5) ein größeres Lumen aufweist als das Lumen (8) des Schaftteilstücks (4).

7. Einführsystem nach einem der Ansprüche 1 bis 6, **dadurch gekennzeichnet, dass** der Prothesenbehälter (5) länger als die Prothese ist.

8. Einführsystem nach einem der Ansprüche 1 bis 7, **dadurch gekennzeichnet, dass** der Prothesenbehälter (5) 5 bis 150 mm länger ist als die Prothese (11).

9. Einführsystem nach einem der Ansprüche 1 bis 8, **dadurch gekennzeichnet, dass** das Druckglied (9) einen Schaft aus einer spiralförmig gewundenen multifilen Reihe an Schaftdrähten (14) aufweist.

10. Einführsystem nach einem der Ansprüche 1 bis 9, **dadurch gekennzeichnet, dass** die Drähte in der ersten Reihe an Drähten (7) und möglicherweise auch in der zweiten Reihe an Drähten oder in der Reihe an Schaftdrähten (14) einen Steigungswinkel im Bereich von 26 bis 76° aufweisen.

11. Einführsystem nach Anspruch 10, **dadurch gekennzeichnet, dass** die Drähte in der ersten Reihe an Drähten (7) und möglicherweise auch in der zweiten Reihe an Drähten oder in der Reihe an Schaftdrähten (14) einen Steigungswinkel im Bereich von 40 bis 65° aufweisen.

12. Einführsystem nach einem der Ansprüche 1 bis 11, **dadurch gekennzeichnet, dass** die Reihe oder die Reihen an Drähten (7, 14) aus einer oder mehreren Gruppen aus vier bis acht spiralförmig gewundener Drähte besteht.

13. Einführsystem nach Anspruch 12, **dadurch gekennzeichnet, dass** die Drähte in einer Gruppe nahe beieinander angeordnet vorliegen.

14. Einführsystem nach einem der Ansprüche 1 bis 13, **dadurch gekennzeichnet, dass** die Drähte in der ersten Reihe in einem Bereich der Einführvorrichtung einen geringeren äußeren Durchmesser aufweisen.

15. Einführsystem nach Anspruch 14, **dadurch gekennzeichnet, dass** der Bereich ein Distalbereich in Kegelform mit in Distalrichtung verringertem äußerem Durchmesser ist.

16. Einführsystem nach einem der vorangegangenen Ansprüche, **dadurch gekennzeichnet, dass** die Distalspitze (3) des Einführsystems mit Mittel zum Markieren (17) ausgestattet ist, um es strahlungsundurchlässig zu machen.

17. Einführsystem nach einem der Ansprüche 1 bis 16, **dadurch gekennzeichnet, dass** das Druckglied (9) mit einer Beschichtung zur Reibungsminderung zwischen dem Druckglied (9) und dem Einführsystem, und / oder die innere Oberfläche des Schaftteilstückes (4) des Einführsystems mit einer Beschichtung zur Reibungsminderung und / oder die innere Oberfläche des Prothesenbehälters (5) mit einer solchen Beschichtung ausgestattet ist.

18. Einführsystem nach einem der Ansprüche 1 bis 17, **dadurch gekennzeichnet, dass** das Einführsystem zumindest in einer 30 cm langen Distalfläche einen maximalen Außendurchmesser von weniger als 2,0 mm aufweist.

19. Einführsystem nach einem der Ansprüche 1 bis 18, **dadurch gekennzeichnet, dass** das Einführsystem zumindest in einer 30 cm langen Distalfläche einen maximalen Außendurchmesser von weniger als 1,00 mm aufweist.

20. Einführsystem nach Anspruche 18 oder 19, **dadurch gekennzeichnet, dass** das Einführsystem in einer Distalendfläche mit einer Länge von zumindest 10 cm einen maximalen äußeren Durchmesser von weniger als 0,30 mm aufweist.

21. Einführsystem nach einem der vorangegangenen Ansprüche, **dadurch gekennzeichnet, dass** die Anzahl der Drähte in der ersten spiralförmig gewundenen Reihe an Drähten (7) entlang der Länge der Einführvorrichtung variiert.

22. Einführsystem nach einem der Ansprüche 4 bis 21, **dadurch gekennzeichnet, dass** die Anzahl der Drähte in der zweiten spiralförmig gewundenen Reihe an Drähten entlang der Länge der Einführvorrichtung variiert.

23. Einführsystem nach einem der vorangegangenen Ansprüche, **dadurch gekennzeichnet, dass** in einem Proximalsegment des Schaftes (4) die erste spiralförmig gewundene Reihe an Drähten (7) durch ein zusätzliches röhrenförmigen Glied versteift wird.

## Revendications

1. Système d'introduction (1) comprenant un dispositif d'introduction (2) présentant une extrémité distale (3) et une portion de tige (4) avec une lumière (8) s'étendant dans la direction longitudinale à partir d'une extrémité proximale vers l'extrémité distale (3), une prothèse auto-expansible (11) arrangée dans un réceptacle de prothèse (5) à l'extrémité distale du dispositif d'introduction, et un élément pousseur (9) arrangé dans la lumière (8) dudit dispositif d'introduction, **caractérisé en ce que** la portion de tige (4) dudit dispositif d'introduction est faite d'une première rangée multifilaire d'au moins deux fils (7) enroulés hélicoïdalement placés l'un à côté de l'autre et aptes à transférer le couple de torsion jusqu'à l'extrémité distale (3) du dispositif d'introduction.

2. Système d'introduction selon la revendication 1, **caractérisé en ce que** ledit réceptacle de prothèse (5) est fixé à ladite première rangée multifilaire de fils (7) enroulée hélicoïdalement dans le prolongement axial de celle-ci.

3. Système d'introduction selon la revendication 2, **caractérisé en ce que** le réceptacle de prothèse (5) est un segment tubulaire de construction multifilaire enroulée.

4. Système d'introduction selon la revendication 3, **caractérisé en ce que** le réceptacle de prothèse (5) est une deuxième rangée multifilaire de fils enroulée hélicoïdalement.

5. Système d'introduction selon la revendication 1, **caractérisé en ce que** le réceptacle de prothèse (5) est un segment distal (13) de ladite première rangée multifilaire de fils (7) enroulée hélicoïdalement.

6. Système d'introduction selon l'une quelconque des revendications 1 à 5, **caractérisé en ce que** le réceptacle de prothèse (5) présente une lumière plus grande que la lumière (8) de ladite portion de tige (4).

7. Système d'introduction selon l'une quelconque des revendications 1 à 6, **caractérisé en ce que** le réceptacle de prothèse (5) est plus long que la prothèse.

8. Système d'introduction selon l'une quelconque des revendications 1 à 7, **caractérisé en ce que** le réceptacle de prothèse (5) est de 5 à 150 mm plus long que la prothèse (11).

9. Système d'introduction selon l'une quelconque des revendications 1 à 8, **caractérisé en ce que** l'élément pousseur (9) présente une tige faite d'une rangée multifilaire de fils de tige (14) enroulée hélicoïdalement.

10. Système d'introduction selon l'une quelconque des revendications 1 à 9, **caractérisé en ce que** les fils dans ladite première rangée de fils (7) et éventuellement aussi dans ladite deuxième rangée de fils ou dans ladite rangée de fils de tige (14) présentent un angle d'hélice compris dans l'intervalle de 26° à 76°.

11. Système d'introduction selon la revendication 10, **caractérisé en ce que** les fils dans ladite première rangée de fils (7) et éventuellement aussi dans ladite deuxième rangée de fils ou dans ladite rangée de fils de tige (14) présentent un angle d'hélice compris dans l'intervalle de 40° à 65°.

12. Système d'introduction selon l'une quelconque des revendications 1 à 11, **caractérisé en ce que** la rangée ou les rangées de fils (7, 14) est/sont faite(s) d'un ou de plusieurs groupes comprenant de 4 à 8 fils enroulés hélicoïdalement.

13. Système d'introduction selon la revendication 12, **caractérisé en ce que** les fils dans un groupe sont situés les uns à côté des autres de manière très rapprochée.

14. Système d'introduction selon l'une quelconque des revendications 1 à 13, **caractérisé en ce que** les fils dans ladite première rangée (7) présentent un diamètre extérieur plus petit à une région du dispositif d'introduction.

15. Système d'introduction selon la revendication 14, **caractérisé en ce que** ladite région est une région distale présentant une forme pointue avec un diamètre extérieur décroissant dans la direction distale.

16. Système d'introduction selon l'une quelconque des revendications précédentes, **caractérisé en ce que** le bout distal (3) du système d'introduction est pourvu d'un moyen de marquage (17) pour le rendre radiopaque.

17. Système d'introduction selon l'une quelconque des revendications 1 à 16, **caractérisé en ce que** l'élément pousseur (9) est pourvu d'un revêtement pour diminuer la friction entre l'élément pousseur (9) et le dispositif d'introduction, et/ou la surface interne de la portion de tige (4) du dispositif d'introduction est pourvue d'un revêtement pour diminuer la friction et/ou la surface interne du réceptacle de prothèse (5) est pourvue d'un tel revêtement.

18. Système d'introduction selon l'une quelconque des revendications 1 à 17, **caractérisé en ce que** le système d'introduction, au moins dans une zone distale de 30 cm de longueur, présente un diamètre extérieur maximum inférieur à 2,0 mm.

19. Système d'introduction selon l'une quelconque des revendications 1 à 18, **caractérisé en ce que** le système d'introduction, au moins dans une zone distale de 30 cm de longueur, présente un diamètre extérieur maximum inférieur à 1,00 mm.

20. Système d'introduction selon la revendication 18 ou 19, **caractérisé en ce que** le système d'introduction, dans une zone d'extrémité distale présentant une longueur d'au moins 10 cm, présente un diamètre extérieur maximum inférieur à 0,30 mm.

21. Système d'introduction selon l'une quelconque des revendications précédentes, **caractérisé en ce que** le nombre de fils dans ladite première rangée de fils (7) enroulée hélicoïdalement varie au long de la longueur du dispositif d'introduction.

22. Système d'introduction selon l'une quelconque des revendications 4 à 21, **caractérisé en ce que** le nombre de fils dans ladite deuxième rangée de fils enroulée hélicoïdalement varie au long de la longueur du dispositif d'introduction.

23. Système d'introduction selon l'une quelconque des revendications précédentes, **caractérisé en ce que**, dans un segment proximal de ladite tige (4), la première rangée de fils (7) enroulée hélicoïdalement est renforcée par un élément tubulaire supplémentaire.
